(19) Europäisches Patentamt
European Patent Office
Office européen des brevets

(11) EP 1 031 350 A1

(12) **EUROPEAN PATENT APPLICATION**

(43) Date of publication:
**30.08.2000 Bulletin 2000/35**

(51) Int Cl.7: **A61K 31/195**

(21) Application number: **99400440.6**

(22) Date of filing: **23.02.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **WARNER-LAMBERT COMPANY**
**Morris Plains New Jersey 07950 (US)**

(72) Inventors:
- **Bryans, Justin Stephen**
  **Balsham CB1 6DZ (GB)**
- **Diop, Laurent**
  **Val d'Albian, 91400 Saclay (FR)**

(74) Representative: **Phélip, Bruno et al**
**c/o Cabinet Harlé & Phélip**
**7, rue de Madrid**
**75008 Paris (FR)**

(54) **Use of a gabapentin-analog for the manufacture of a medicament for preventing and treating visceral pain**

(57) [1S-(1α,3β)]-(1-aminomethyl-3-methyl-cyclohexyl)-acetic acid is useful to prevent and treat visceral pain.

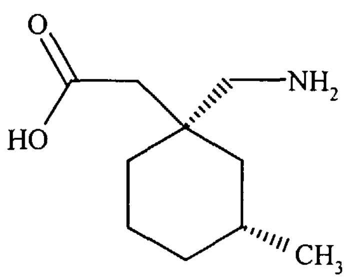

EP 1 031 350 A1

## Description

FIELD OF THE INVENTION

This invention relates to a method for preventing and for treating visceral pain, and gastrointestinal disorders such as functional bowel disorders (FBD) and inflammatory bowel diseases (IBD) through the use of effective amounts of [1S-(1α, 3β)]-(1-aminomethyl-3-methyl-cyclohexyl)-acetic acid.

BACKGROUND OF THE INVENTION

Gabapentin (1-(aminomethyl)cyclohexane acetic acid) is an antiepileptic drug, active in various animal models of epilepsy, and effective in decreasing the frequency of seizures in patients. Gabapentin, although a γ-aminobutyric acid (GABA) structural analogue, does not significantly bind to GABA receptors, but binds to the $\alpha_2\delta$ subunit of a calcium channel (Gee N.S. *et al*. (1996) J. Biological Chemistry **271**: 5768-5776), suggesting that modulation of voltage-dependent neuronal $Ca^{2+}$ channels is important to the action of gabapentin. Also, a correlation has been shown between the affinity of ligands at the [$^3$H]gabapentin binding site and anticonvulsant activity (Taylor C. P. *et* al. (1993) Epilepsy Res. **14**: 11-15). Gabapentin s.c. has recently been shown to suppress TNBS-induced colonic hypersensitivity in a model of chronic allodynia (Diop L. *et al*. (1998) Soc. Neurosci. Abstr.: **24**: 639).

International patent application WO 97/33858 describes substituted derivatives of gabapentin, including [1S-(1α, 3β)]-(1-aminomethyl-3-methyl-cyclohexyl)-acetic acid, which bind to the $\alpha_2\delta$ subunit of a calcium channel, and are useful in the treatment of epilepsy and related central nervous system (CNS) disorders.

Commonly encountered gastrointestinal (GI) disorders include the functional bowel disorders (FBD) and the inflammatory bowel diseases (IBD). These GI disorders include a wide range of disease states that are currently only moderately controlled, including - for FBD, gastro-esophageal reflux, dyspepsia, and the irritable bowel syndrome (IBS), and - for IBD, Crohn's disease, ileitis, and ulcerative colitis, and that all regularly produce visceral pain. It has been shown recently in these pathologies, in particular the irritable bowel syndrome and dyspepsia, that the visceral pain threshold is decreased, indicating a visceral hypersensitivity.

Few drugs are known to act selectively upon GI disorder-associated hypersensitivity (Farthing M.J. (1998) Drugs **56**:11-21).

Available treatments of pain fall into two categories: 1) nonsteroidal antiinflammatory drugs, used to treat mild pain, but whose therapeutic use is limited by GI adverse effects (gastric erosion, peptic ulcer formation, inflammation of the duodenum and colon); 2) morphine and related opioids, used to treat moderate to severe pain but whose therapeutic use is limited by undesirable side effects including constipation, respiratory depression, tolerance, and abuse potential.

There is a need for drugs that can alleviate visceral pain without undesirable side effects.

SUMMARY OF THE INVENTION

This invention provides a method for preventing and treating visceral pain and GI disorders comprising administering to a subject in need of treatment an effective amount of [1S-(1α,3β)]-(1-aminomethyl-3-methyl-cyclohexyl)-acetic acid (I)

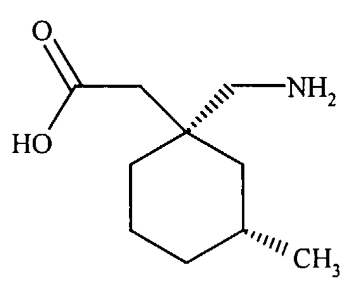

**(I)**

or a pharmaceutically acceptable salt thereof.
This invention also concerns the use of a compound of Formula I for the preparation of a medicament useful for preventing or treating visceral pain and gastrointestinal disorders, in particular by the oral route.

DETAILED DESCRIPTION OF THE INVENTION

The compound used in the instant invention, [1S-(1α, 3β)]-(1-aminomethyl-3-methyl-cyclohexyl)-acetic acid (Formula I above), is described in international patent application WO 97/33858 which is incorporated herein by reference, and which more generally describes substituted derivatives of gabapentin, including [1S-(1α, 3β)]-(1-aminomethyl-3-methyl-cyclohexyl)-acetic acid; such derivatives bind to the $\alpha_2\delta$ subunit of a calcium channel, and are useful in the treatment of epilepsy. In that application WO 97/33858, the compound utilized in the instant invention has been shown to have an affinity similar to that of gabapentin for the $\alpha_2\delta$ subunit derived from porcine brain tissue.

It has now been found that [IS-(la, 3 (3)]-( 1-aminomethyl-3-methyl-cyclohexyl)-acetic acid, although of similar activity or less active than gabapentin on somatic pain or epilepsy, is surprisingly ten-fold more effective than gabapentin on visceral pain.

This finding is at odds with the well recognized knowledge that, at every level of the nervous system, a close relationship prevails between somatic pain pathways and visceral pathways (Cross S.A. (1994) Mayo Clin Proc **69**: 375-83).

The compounds utilized in the present invention include solvates, hydrates, pharmaceutically acceptable salts, and polymorphs (different crystalline lattice descriptors) of the compound of Formula I.

Where it is appropriate to form a salt, the pharmaceutically acceptable salts include acetate, benzenesulfonate, benzoate, bitartrate, calcium acetate, camsylate, carbonate, citrate, edetate, edisylate, estolate, esylate, fumarate, gluceptate, gluconate, glutamate, glycoloylarsanilate, hexylresorcinate, hydrabamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemisulfate, tannate, tartrate or hemi-tartrate, theoclate, triethiodide, benzathine, chloroprocaine, choline, diethanolamine, ethylenediamine, meglumine, procaine, aluminum, ammonium, tetramethyl ammonium, calcium, lithium, magnesium, potassium, sodium, and zinc. (See also "Pharmaceutical salts" by Berge S.M. *et al.* (1997) J. Pharm. Sci. **66**: 1-19, which is incorporated herein by reference.)

The term "patient" is intended to include a mammal, especially a human.

All that is required to practice the method of preventing and treating visceral pain and GI disorders FBD or IBD according to the present invention is to administer [1S-(1α,3β)]-(1-aminomethyl-3-methyl-cyclohexyl)-acetic acid in an amount that is effective to prevent or treat the damaged condition, i.e. to control visceral pain and/or FBD or IBD. The effective amount of [1S-(1α,3β)]-(1-aminomethyl-3-methyl-cyclohexyl)-acetic acid to be utilized will generally be from about 1 to about 300 mg / kg of patient body weight. Typical doses will be from about 10 to about 5000 mg per day for an adult patient of normal weight. Typical FBD conditions include gastro-esophageal reflux disease, dyspepsia, and IBS. Typical IBD conditions include ileitis, ulcerative colitis, and Crohn's disease.

In a further aspect of the present invention, there is provided a pharmaceutical composition for the treatment or prevention of visceral pain and GI disorders comprising the active component, [1S-(1α, 3β)]-(1-aminomethyl-3-methyl-cyclohexyl)-acetic acid, of Formula I. Pharmaceutical compositions of the compound of the present invention -including one of its salts, are produced by formulating this active component in dosage unit form with at least one pharmaceutically acceptable carrier or excipient. For preparing pharmaceutical compositions from the compound used in this invention, inert, pharmaceutically acceptable carriers can be either solid or liquid.

Solid dosage forms for oral administration include capsules, tablets, pills, powders, and granules. They preferably contain 5% to about 70% of [1S-(1α, 3β)]-(1-aminomethyl-3-methyl-cyclohexyl)-acetic acid. In such solid dosage forms, the active component is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alginates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose as well as high molecular weight polyethyleneglycols, and the like.

Solid dosage forms such as tablets, dragées, capsules, pills, and granules can be prepared with coatings and shells, such as enteric coatings and others well known in the art. They can also be of such composition that they release the active component in a certain part of the intestinal tract in a delayed manner. Examples of embedding compositions

which can be used are polymeric substances and waxes. The active component can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to [1S-(1α, 3β)]-(1-aminomethyl-3-methyl-cyclohexyl)-acetic acid, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, and the like.

Suspensions, in addition to the active component, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compound of the present invention with suitable non-irritating excipients or carriers such as cocoa butter, polyethyleneglycol or a suppository wax, which are solid at ordinary temperatures but liquid at body temperature, and therefore melt in the rectum and release the active component.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable liquid carriers, diluents, solvents or vehicles include water, ethanol, polyols (propyleneglycol, polyethyleneglycol, glycerol, and the like), and suitable mixtures thereof.

These compositions may also contain adjuvants such as preserving, wetting, emulsifying, and dispersing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like.

Preferably the pharmaceutical preparation is in unit dosage form. In such form, the preparation is divided into unit doses containing appropriate quantities of [1S-(1α,3β)]-(1-aminomethyl-3-methyl-cyclohexyl)-acetic acid. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms. Some examples of dosage unit forms are tablets, capsules, pills, powders, suppositories, aqueous and nonaqueous oral solutions and suspensions, and parenteral solutions packaged in containers containing either one or some larger number of dosage units and capable of being subdivided into individual doses.

The percentage of the active component in the foregoing compositions can be varied within wide limits, but for practical purposes it is preferably present in a concentration of at least 10 % in a solid composition and at least 2 % in a primary liquid composition. The most satisfactory compositions are those in which a much higher proportion of the active component is present, for example, from 10 % to 90 % by weight.

Routes of administration of [1S-(1α,3β)]-(1-aminomethyl-3-methyl-cyclohexyl)-acetic acid or its salts are parenteral or, preferably, oral. For example, a useful oral dosage is between 20 and 800 mg, and a useful intravenous dose is between 5 and 50 mg. The dosage is within the dosing range used in treatment of visceral pain and GI disorders such as FBD or IBD, or as would be dictated by the needs of the patient as described by the physician.

A unit dosage form of [1S-(1α, 3β)]-(1-aminomethyl-3-methyl-cyclohexyl)-acetic acid to be used in this invention may also comprise other compounds useful in the therapy of visceral pain and GI disorders.

The advantages of using [1S-(1α, 3β)]-(1-aminomethyl-3-methyl-cyclohexyl)-acetic acid in the instant invention include the selective activity of the compound on visceral pain, the relatively nontoxic nature of the compound, the ease of preparation, the fact that the compound is well tolerated, and the ease of i.v. and, in particular, oral administration of the drug.

<u>Pharmacological data</u>

Initially, [1S-(1α, 3β)] -(1-aminomethyl-3-methyl-cyclohexyl)-acetic had proved of similar activity or less active than gabapentin in animal models of somatic pain and epilepsy. However, as regards visceral pain, the ability of [1S-(1α, 3β)]-(1-aminomethyl-3-methyl-cyclohexyl)-acetic acid to treat selectively GI disorders according to this invention has been established in an animal model of allodynia.

**<u>Somatic pain:</u>**

<u>Carrageenin-induced hyperalgesia in rats: effect of gabapentin and [1S-(1α, 3β)]-(1-aminomethyl-3-methyl-cyclohexyl)-acetic acid</u>

Nociceptive pressure thresholds were measured in the rat paw pressure test using an analgesymeter (Randall

L.O. and Sellitto J.J. (1957) Arch. Int. Pharmacodyn. **4**: 409-419). Male Sprague Dawley rats (70-90 g) were trained on this apparatus before the test day. Pressure was gradually applied to the hind paw of each rat and nociceptive thresholds were determined as the pressure (g) required to elicit paw withdrawal. A cutoff point of 250 g was used to prevent any tissue damage to the paw. On the test day, two to three baseline measurements were taken before animals were administered 100 μl of 2% carrageenin by intraplantar injection into the right hind paw. Nociceptive thresholds were taken again 3 h after carrageenin to establish that animals were exhibiting hyperalgesia. Animals were dosed with either gabapentin, [1S-(1α, 3β)]-(1-aminomethyl-3-methyl-cyclohexyl)-acetic acid, or saline at 3.5 h after carrageenin, and nociceptive thresholds were examined at 4, 4.5, and 5 h post-carrageenin.

Results

Effect of gabapentin

| ***Dose p.o.*** **(mg/kg)** | **Inhibition at 1 h (%)** | **Inhibition at 2 h (%)** |
|---|---|---|
| 30 | 48 | 20 |

Effect of [1S-(1α, 3β)]-(1-aminomethyl-3-methyl-cyclohexyl)-acetic acid:

| **Dose *p.o.* (mg/kg)** | **Inhibition at 1 h (%)** | **Inhibition at 2 h (%)** |
|---|---|---|
| 30 | 68 | 34 |

**Epilepsy:**

Semicarbazide-induced tonic seizures in mice: effect of gabapentin and [1S-(1α, 3β)]-(1-aminomethyl-3-methyl-cyclohexyl)-acetic acid

Tonic seizures in mice are induced by subcutaneous administration of semicarbazide (750 mg/kg). The latency to the tonic extension of forepaws is noted. Any mice not convulsing within 2 h after semicarbazide are considered protected and given a maximum latency score of 120 min.
At the same dose of 30 mg/kg *p.o.,* gabapentin protected 100% of the animals whereas [1S-(1α, 3β)]-(1-aminomethyl-3-methyl-cyclohexyl)-acetic acid protected 40%.

**Visceral pain:**

TNBS-induced chronic visceral allodynia in rats: effect of gabapentin and [1S-(1α, 3β)]-(1-aminomethyl-3-methyl-cyclohexyl)-acetic acid

Injections of trinitrobenzene sulfonic acid (TNBS) into the wall of the rat colon have been found to induce chronic colitis. In humans, GI disorders are often associated with visceral pain. In these pathologies, the visceral pain threshold is decreased indicating a visceral hypersensitivity. This study was designed to evaluate the effect of [1S-(1α, 3β)]-(1-aminomethyl-3-methyl-cyclohexyl)-acetic acid *p.o.* in an experimental model of colonic distension in awake rats; previous injection of TNBS into the proximal colon of the rats had lowered their visceral pain threshold. This effect is compared with that of gabapentin *p.o.*

Materials and methods

Male Sprague-Dawley rats weighing 340-400 g are used. The animals are housed 3 per cage in a regulated environment (20 ± 1°C, 50 ± 5 % humidity, with light 8:00 am to 8:00 pm). At day 0, under anesthesia (ketamine 80 mg/kg *i.p.;* acepromazine 12 mg/kg *i.p.*), the injection of TNBS (50 mg/kg in ethanol 30 %), or saline (1.5 ml/kg) for control rats, is performed into the proximal colon wall (1 cm from the cecum). After the surgery, animals are individually housed in polypropylene cages and kept in a regulated environment (20 °C, 50 % humidity, with light 8:00 a.m. to 8:00 p.m.) during 7 days. At day 7 after TNBS administration, a balloon (5-6 cm length) is inserted by anus, and kept in position (tip of balloon 5 cm from the anus) by taping the catheter to the base of the tail. Oral administration of gabapentin or of [1S-(1α, 3β)]-(1-aminomethyl-3-methyl-cyclohexyl)-acetic acid is performed 1 h before the colonic distension cycle: the balloon is progressively inflated by steps of 5 mm Hg (0.667 kPa), from 0 to 75 mm Hg, each step of inflation lasting 30 s. Each cycle of colonic distension is controlled by a standard barostat. The threshold (mm Hg) corresponds

to the pressure which produced the first abdominal contraction, and the cycle of distension is then discontinued. The colonic threshold is determined after performance of four cycles of distension on the same animal.

Data is analyzed by comparing test compound-treated groups with a TNBS only-treated group and the control group. Mean and SEM are calculated for each group. The antiallodynic activity of each oral dose of the test compound is calculated as follows:

$$Activity = \frac{A-T}{C-T}$$

where

A = mean threshold of the test compound-treated group,
T = mean threshold of the TNBS only-treated group,
C = mean threshold of the control group.

The results measured in the test compound-treated groups are expressed in % inhibition of the TNBS-induced decrease in the pain threshold.

Statistical significance between each group was determined by using a one-way ANOVA followed by Student's unpaired *t*-test; differences were considered statistically significant at $p < 0.05$.

Results

Effect of gabapentin:

| Dose *p.o.* (mg/kg) | Inhibition at 1 h (%) | Number of rats |
|---|---|---|
| 100 | 14.7 ± 5.1 %* | 7 |
| 300 | 48.6 ± 13.3 %** | 8 |
| 500 | 64.9 ± 10.5 %*** | 8 |
| 1000 | 75.2 ± 6.1 % *** | 8 |

* : p < 0.05
** : p < 0.01
*** : p < 0.001

The median effective dose ($ED_{50}$) of gabapentin is 321 mg/kg *p.o.*

Effect of [1S-(1α, 3β)]-(1-aminomethyl-3-methyl-cyclohexyl)-acetic acid:

| Dose *p.o.* (mg/kg) | Inhibition at 1 h (%) | Number of rats |
|---|---|---|
| 10 | 34.0 ± 9.7 % * | 8 |
| 30 | 46.6 ± 8.4 % ** | 7 |
| 100 | 97.4 ± 22.4 % ** | 8 |

The $ED_{50}$ of [1S-(1α, 3β)]-(1-aminomethyl-3-methyl-cyclohexyl)-acetic acid is 22.9 mg/kg *p.o.*

By the subcutaneous *(s.c.)* route, gabapentin is known not to modify the colonic threshold in control conditions; by contrast, in the same conditions morphine *(s.c.)* increased the colonic threshold in both TNBS-treated animals and in controls, suggesting a different mechanism of action. (Diop L. *et al.* (1998) Soc. Neurosci. Abstr.: **24**: 639).

[1S-(1α, 3β)]-(1-aminomethyl-3-methyl-cyclohexyl)-acetic acid produced a potent antiallodynic activity in a model of visceral pain in rats, the compound being more than 10-fold more active than gabapentin in the same conditions. The respective $ED_{50}$ values are 321 mg/kg *p.o.* for gabapentin and 23 mg/kg *p.o.* for [1S-(1α,3β)]-(1-aminomethyl-3-methyl-cyclohexyl)-acetic acid. Furthermore this effect does not involve an opiate mechanism.

The foregoing data establish that [1S-(1α, 3β)]-(1-aminomethyl-3-methylcyclohexyl)-acetic acid is effective in preventing and treating visceral pain, in particular in GI disorders such as functional bowel disorders (FBD) and inflammatory bowel diseases (IBD). In addition, such efficacy of [1S-(1α, 3β)]-(1-aminomethyl-3-methyl-cyclohexyl)-acetic acid is observed following oral administration of the compound.

**Claims**

1. A method for preventing visceral pain comprising administering to a patient in need of treatment an effective amount of [1S-(1α,3β)]-(1-aminomethyl-3-methyl-cyclohexyl)-acetic acid or a pharmaceutically acceptable salt thereof.

2. A method for treating visceral pain comprising administering to a patient in need of treatment an effective amount of [1S-(1α, 3β)]-(1-aminomethyl-3-methyl-cyclohexyl)-acetic acid or a pharmaceutically acceptable salt thereof.

3. A method for preventing gastrointestinal disorders comprising administering to a patient in need of treatment an effective amount of [1S-(1α, 3β)]-(1-aminomethyl-3-methyl-cyclohexyl)-acetic acid or a pharmaceutically acceptable salt thereof.

4. A method for treating gastrointestinal disorders comprising administering to a patient in need of treatment an effective amount of [1S-(1α,3β)]-(1-aminomethyl-3-methyl-cyclohexyl)-acetic acid or a pharmaceutically acceptable salt thereof.

5. A method according to Claim 3 or Claim 4 wherein the gastrointestinal disorder is characterized as functional bowel disorder or inflammatory bowel disease.

6. A method according to Claim 3 or Claim 3 wherein the gastrointestinal disorder is a functional bowel disorder.

7. A method according to Claim 3 or Claim 4 wherein the gastrointestinal disorder is gastro-esophageal reflux disease.

8. A method according to Claim 3 or Claim 4 wherein the gastrointestinal disorder is dyspepsia.

9. A method according to Claim 3 or Claim 4 wherein the gastrointestinal disorder is the irritable bowel syndrome.

10. A method according to Claim 3 or Claim 4 wherein the condition treated is selected from Crohn's disease, ileitis, and ulcerative colitis.

11. A method according to Claim 3 or Claim 4 wherein [1S-(1α,3β)]-(1-aminomethyl-3-methyl-cyclohexyl)-acetic acid or a pharmaceutically acceptable salt thereof is administered by the oral route.

12. The use of [1 S-(1α, 3β)]-(1-aminomethyl-3-methyl-cyclohexyl)-acetic acid or a pharmaceutically acceptable salt thereof for the preparation of a medicament useful for preventing or treating visceral pain.

13. The use according to claim 12 wherein the medicament is a formulation for oral administration.

European Patent Office

# EUROPEAN SEARCH REPORT

Application Number
EP 99 40 0440

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| E | WO 99 08671 A (BUENO LIONEL ;CHOVET MARIA (FR); DIOP LAURENT (FR); LITTLE HILARY) 25 February 1999 (1999-02-25) * page 1 - page 3; claims 1-11 * | 1-13 | A61K31/195 |
| D,Y | WO 97 33858 A (WARNER LAMBERT CO ;HORWELL DAVID CHRISTOPHER (GB); BRYANS JUSTIN S) 18 September 1997 (1997-09-18) * page 1 - page 3 * * page 15, line 25 - line 30 * * page 17, line 1 - line 21 * | 1-13 | |
| Y | WO 96 11680 A (GLAXO GROUP LTD ;BAYS DAVID EDMUND (GB); BOUNTRA CHARANJIT (GB)) 25 April 1996 (1996-04-25) * page 1, line 24 - line 28 * * page 2, line 32 - page 3, line 17 * | 1-13 | |

TECHNICAL FIELDS SEARCHED (Int.Cl.7)
A61K

The present search report has been drawn up for all claims

| Place of search | Date of completion of the search | Examiner |
|---|---|---|
| MUNICH | 12 October 1999 | Tzschoppe, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 40 0440

This annex lists the patent family members relating to the patent documents cited in the above-mentioned European search report. The members are as contained in the European Patent Office EDP file on
The European Patent Office is in no way liable for these particulars which are merely given for the purpose of information.

12-10-1999

| Patent document cited in search report | Publication date | Patent family member(s) | Publication date |
|---|---|---|---|
| WO 9908671 A | 25-02-1999 | AU 8668598 A | 08-03-1999 |
| | | AU 9293098 A | 08-03-1999 |
| | | WO 9908670 A | 25-02-1999 |
| WO 9733858 A | 18-09-1997 | AU 2051197 A | 01-10-1997 |
| | | BG 102733 A | 30-04-1999 |
| | | CA 2244912 A | 18-09-1997 |
| | | CZ 9802863 A | 17-03-1999 |
| | | EP 0888286 A | 07-01-1999 |
| | | NO 984205 A | 14-09-1998 |
| | | PL 328816 A | 15-02-1999 |
| WO 9611680 A | 25-04-1996 | AU 3745895 A | 06-05-1996 |

EPO FORM P0459

For more details about this annex : see Official Journal of the European Patent Office, No. 12/82